# EUROPEAN PATENT APPLICATION

(11) **EP 4 011 354 A1**
(43) Date of publication of application: **15.06.2022**
(21) Application number: 20212807.0
(22) Date of filing: 09.12.2020
(51) Int. Cl.: A61K 8/34, A61K 8/44, A61K 8/60, A61Q 5/00, A61Q 17/04, A61Q 19/00, A61K 8/365, A61K 8/42, A61Q 15/00

(54) **NEW COSMETICS SOLVENTS BASED ON TWO DIFFERENT COMPONENTS**

(71) Applicant: Beiersdorf AG, 20253 Hamburg (DE)
(72) Inventor: Piacentini, Adalberto, 22529 Hamburg (DE); Köhler, Manuela, 22527 Hamburg (DE); Bernau, Mareike, 22087 Hamburg (DE); Schweiger, Dorothea, 22459 Hamburg (DE); Klauck, Robert, 21465 Reinbek (DE); Ferraro, Claudio, 1007 Lausanne (CH); Kühnl,, Jochen, 20249 Hamburg (DE)
(74) Representative: Beiersdorf AG

(57) **Abstract**

The invention is an eutectic solvent which is formed from the mixture of two different components selected from the group of sugars, aminoacids, short organic acids, polyols, betaines, quaternary ammonium salts in precise molar ratio. The new inventive eutectic solvents allow an improved incorporation of active ingredients into cosmetic compositions.

## Description

The invention is an eutectic solvent which is formed from the mixture of two different components selected from the group of sugars, aminoacids, short organic acids, polyols, betaines, quaternary ammonium salts in precise molar ratio.

Natural deep eutectic solvents (NADES) are bio-based ionic liquids and deep eutectic solvents which are composed of two or more compounds that are generally plant based primary metabolites, i.e. organic acids, sugars, alcohols, amines and amino acids. Ionic liquids are salts, liquid at room temperature, characterized by ionic bonds which have at least one large organic ion and a cation with a low degree of symmetry. The positive and negatively charged ions in the liquid are thus kept far apart so that they reduce the attractive forces between them and hinder crystallization, lowering the melting point and resulting in a completely ionic liquid at room temperature. Deep eutectic solvents (DES) are mixtures of solid compounds that form liquids due to a large depression of the melting point; the driving force in the case of DES is the extensive hydrogen bonding between components forming a non-aqueous liquid at room temperature. Work done by Choi, Spronsen *et al.* showed that water can be present as part of the solvent, being strongly retained in the liquid and which cannot be evaporated.

FR 3036618 describes NADES, methods in which these NADES are involved, plant extracts obtained by using the NADES and the use of these plant extracts for the preparation of cosmetic compositions.

The NADES are liquid at room temperature and characterized in that they consist of a first and a second compound, each selected from the group comprising the family of polyols, the family of sugars or the family of amino acids and that the first compound and the second compound are selected from different families, the NADES may advantageously further comprise an aqueous phase.

FR 3017292 describes a cosmetic formulation based on vegetable or natural origin, a cosmetic composition containing the formulation, and a process for the preparation of such a cosmetic composition. It also relates to the use of a eutectic solvent as the basis of a cosmetic formulation and to a device comprising a cosmetic composition. The cosmetic formulation base comprises a eutectic solvent consisting of natural or plant molecules. The mixture of a eutectic solvent and of an active ingredient until a homogeneous dispersion is obtained and the resulting homogeneous dispersion is cooled until a balsam texture is obtained.

FR 3046352 describes the cosmetic use of a eutectic solvent consisting of molecules of vegetable or natural origin, as an agent that enhances the barrier function of the epidermis to improve the radiance of the skin, and / or smooth the skin surface and / or smooth the skin and / or improve the complexion of the skin.

The eutectic solvent comprises at least one active ingredient selected from mangiferin, mangustin, rutin, apigenin, esculin, mannitol, Baaleinin lupeol acetate, coumarins, polyols, triterpenes, saponins, carotenoids, polyphenols or a mixture thereof or plant extracts rich in these molecules, such as an ash manna extract (fraxinus ornus) rich in mannitol, an extract of chicle gum (Manilkara zapota) rich in lupeol acetate, an extract of scutellar (Scutellaria baïcalensis), rich in bayalein or an extract of green coffee beans, which are rich in chlorogenic acids.

The following problems need to be improved or resolved.

Active ingredients are normally incorporated into cosmetic products as a solution in water. However, after application, water evaporates quickly and the actives remaining in solution precipitate. Once the actives are precipitated, they are no longer bioavailable and display no more activity.

Vitamin C (ascorbic acid) is one of the common active ingredients and a compound with proven biological activity for cosmetic application, however it decomposes in presence of water and air, typically in the span of a few days to a few weeks. This behaviour limits its incorporation to cosmetically acceptable and long-term stable formulas.

Recently there has been extensive studies about the possibilities of achieving better skin properties by ensuring the microbiome of the skin is well balanced. One strategy is to deliver living bacteria which display a positive effect on the microbiome. However, it is a challenge to find a suitable medium where the desired bacteria strain remains alive and in the desired concentration, that means no growth or death.

Another problem seems to be that some compounds needed to ensure the functionality of a cosmetic formulation, such as organic UV-filters and preservatives, can penetrate the skin barrier, leading to undesired biological effects. Their activity is only desired on the external surface of the skin - however there should be little possibilities of decreasing their penetration without modifying their chemical structure.

In addition, long-last moisturization of the skin has always being a cosmetic challenge, specially taking sensory of the final formula into consideration. Water-binding substances such as glycerine, propanediol and urea are commonly used, but their alternatives are still relatively scarce. New substances capable of enhancing the perceived moisturization of the skin are needed in order to expand the formulation possibilities.

All these challenges and problems are solved by the inventive eutectic solvent or by a composition, especially cosmetic composition, comprising such an eutectic solvent, which is formed from the mixture of two different components selected from the group consisting of Alanine, Betaine, Choline chloride, Citric acid, Glycerol, Malic acid, Trehalose, Urea, Glucose, 1,3 - Propanediol, Xylitol, Proline, Sorbitol and Glycine, wherein the molar ratio of the two components being preferably chosen in the range from 1 to 4 to 4 to 1.

Preferably the eutectic solvent comprises water in addition to an amount up to 40 %, preferably up to 20 % by weight based on the total mass of the eutectic solvent and water.

The inventive eutectic solvents or a composition comprising the eutectic solvents shows
- a new way to increase the availability of actives by solving them in new hydrophilic solvents which do not evaporate;
- a surprising new way of delivering sensitive actives, e.g. myriceline or ascorbic acid, in a liquid form with reduced degradation compared to water solution;
- a remarkable boost and long-lasting moisturization when compared to usual cosmetic strategies;
- a new way of blocking sweat glands via the water triggered precipitation of water-insoluble salts dissolved in new solvents;
- a new hydrophilic bacteriostatic solvent, in which gram positive and gram-negative bacteria can remain dormant and be reactivate by simple dilution in water;
- a new solvent, suitable for AP actives e.g. ACH, Magnesium salts, etc. and boosting its anti-transpirant properties when compared to a water solution;
- a solvent that hinders the penetration of cosmetic compounds which activity is expected on the surface of the skin and dermal absorption is not desired (e.g. UV-filters and preservatives)

Surprisingly, these new class of solvents was developed by combining two substances selected from the group of sugars, aminoacids, short organic acids, polyols, betaines, quaternary ammonium salts in precise molar ratio leading to the formation of a liquid phase displaying a melting point lower than the individual compounds. In most of the cases, the new liquids do not crystallize as pure substances does, but rather become amorphous solids upon cooling. Therefor no melting point can be determined experimentally, only a glass transition temperature. But these new solvents display a negligible vapor pressure, therefore they do not evaporate at room temperature and pressure.

The new inventive eutectic solvents allow an improved incorporation of active ingredients into cosmetic compositions. The active ingredient is stabilized and more readily available. Therefore, the amount of active ingredients can be reduced in the composition without of an decrease of activity.

As preferred the following active ingredients could be added to the inventive composition: Glycyrrhiza Inflata Root Extract, Silver Citrate, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecene Dioic acid, Caffeine, Carnitine, Creatine, Cystine, I-tyrosine, Alpha-Glucosylrutin, Taurine, Vitamin C/Ascorbic acid, Glycyrrhizic acid, N-Acetylhydroxyprolin, Niacinamide, Magnesium-Ascorbylphosphat, Ellagic acid, licorice root extract, sea salt, Isoserinol, Ascorbylglucosid, Undecenoylphenylalanin, Kojic acid, arbutin, Zingerone, Dihydromyricetin, 4-Hexyl-Resorcin, Phenylethyl Resorcinol, Ubiquinone, esp. Q10, Cyanomethylphenyl Menthan Carboxamide, Menthoxypropanediol, Menthan Carboxamid Ethylpyridin, Hydroxyethylurea, Climbazole, Epsilon-Poly-L-Lysine, Laureth-9, Piroctone Olamine, Selenium sulfide, zinc pyrithione as well as deodorant- and/or antiperspirant active ingredients and as well as plant- and/or fruit extracts.

The solubility of actives is higher in inventive eutectic solvents than in water and contrarily to oils, these solvents are hydrophilic and have the same (or even superior) capacity to dissolve actives, like folic acid, Thiamidol or even other molecules like tapioca starch.

Water in contact with these liquids is often in a remarkably bound state due to extensive hydrogen bonding interactions. Some of these liquids integrated into cosmetic formulas lead to an improved moisturization of the skin due to extensive water binding.

Surprisingly, the following advantages based on the eutectic solvents according to the invention, which are explained in more detail below.

The combination of the solid ascorbic acid with the inventive eutectic solvents surprisingly yields a liquid due to the decrease in the melting point. This liquid, stable at room temperature, is water-free and can be incorporated into cosmetic formulas. Furthermore, it was shown that the addition of water up to 40% can be used as a strategy to decrease the viscosity of the liquid without increased degradation of the ascorbic acid. This lead to decreased vitamin C degradation compared to water solutions used nowadays.

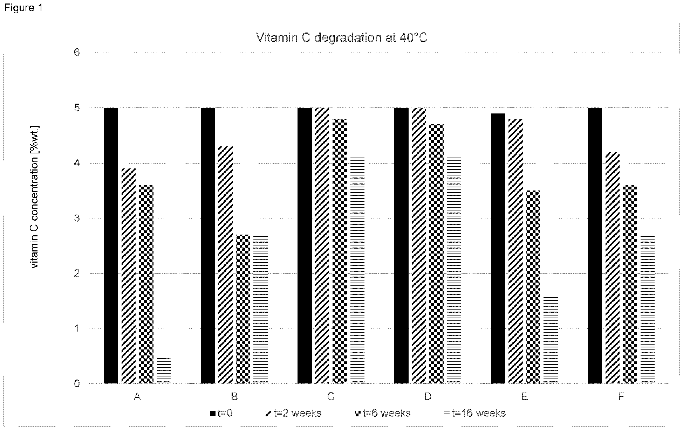

As an example of decreased degradation of vitamin C dissolved in inventive eutectic solvents compared to water, the stability of 5 % vitamin C solutions was followed at 40 degrees for 16 weeks, leading to a surprisingly enhanced protection of vitamin C in inventive eutectic solvents, as shown in figure 1.

The tests show that only 10% of the initial vitamin C was still measurable in the aqueous solution after 16 weeks but up to 82% of the active was still intact in the eutectic solvents.

**table 1: Molar ratios of each eutectic solvent of diagram 1, in which 5% wt. vitamin C was added:**

| | **solvent (NADES)** | **Ratio (mol:mol)** | **Water content** |
|---|---|---|---|
| A | Water | -- | 100% |
| B | Urea + Choline chloride | 2:1 | 0% |
| C | Betaine + Glycerol | 1:3 | 5% |
| D | Betaine + Trehalose | 4:1 | 20% |
| E | Betaine + Urea | 2:3 | 10% |
| F | Betaine + Malic Acid | 1:1 | 20% |

Some water-insoluble compounds such as uric acid, calcium lactate and l-tyrosine can be dissolved in higher concentration into the created inventive eutectic solvents. When this composition comes in contact with water from sweat in the pores, the compounds precipitate, thus blocking the sweat flow.

Therefor a new antiperspirant composition is developed based on the eutectic solvents according to the invention.

This invention consists in the identification of water-insoluble salts which solubility would be increased in inventive eutectic solvents.

In a second step, due to dilution caused by sweating, the solubility of the salt would tend to decrease, leading to precipitation and blocks the sweat glands.

As an example, the following compounds were tested in eutectic solvents:
Calcium carbonate, calcium hydroxide, magnesium carbonate, uric acid, calcium lactate, l-cystine and l-tyrosine. Positive hits were identified, with the surprisingly high solubility of this compounds, esp. calcium lactate, in Betaine : Glycerol (ratios 1:3 and 1:4) eutectic solvent, when compared to pure water.

The concept was tried and successfully blocked a 50 micrometer channel during an *in vitro* test, performed using a microfluidic device. In such device, a flow of artificial sweat is mixed with a flow of the test formula, and the blocking of the channel is evaluated 24h later.

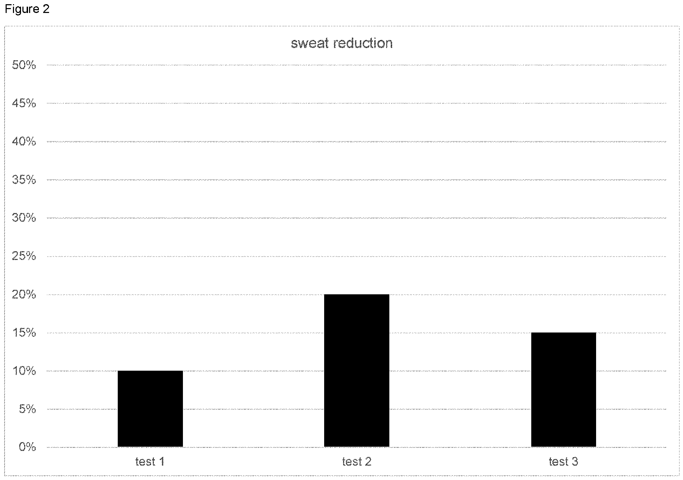

In addition, a sweat reduction test in vivo with Calcium Lactate was carried out. The figure 2 shows in vivo tests showing sweat reduction for Betaine-Glycerine (molar ratios 1:3) and 10,5 wt.% calcium lactate and 37,5 wt.% water.

When one or more of the initial compounds are solid at room temperature, it is possible to trigger its crystallization - and if this solid formation happens preferentially inside the sweat gland pores, anti-perspirant activity is observed.

The efficacy of aluminium comprising antitranspirant products depends heavily on the aggregation of aluminium salts, which can be further improved by the right choice of the solvent, leading to a boost in efficacy. The anti-transpirant efficacy of aluminium salts is surprisingly boosted when dissolved in the inventive solvents.

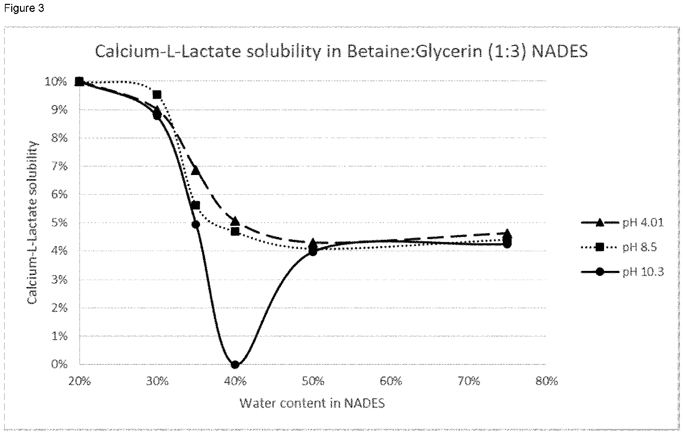

Figure 3 shows an example of the study of the calcium lactate solubility in Betaine decreasing with water at different pH.

It shows that the addition of water leads to a decrease into salt solubility. This happens at low, medium and basic pH.

This is the surprising behavior that leads to the invention of using the decrease in salt solubility as the water content increases as the basis to use the sweat to trigger precipitation.

Also, the inventive solvent, when liquid at room temperature, can be used for suspending bacteria. Surprisingly, the bacteria are kept in a dormant and no bacterial growth or death takes place, due to the absence of water, or its low activity when only present up to 50% wt.

The combination of two of the inventive compounds lead to a mixture displaying a melting point significantly inferior to the individual compounds and liquid at room temperature. The mechanism behind is related to a strong hydrogen-bonding capacity between the molecules. This strong molecular interaction can happen also with molecules solubilized in the mentioned liquid, leading to a decrease in the liberation and dermal-absorption of the compound during pig-skin penetration test.

Actives liberation and dermal penetration behavior were evaluated for a 0,5% caffeine solution in an inventive eutectic solvent, betaine : malic acid (1:1 + 20% water; NADES). Caffeine is chosen as a model active molecule with an important measured dermal absorption.

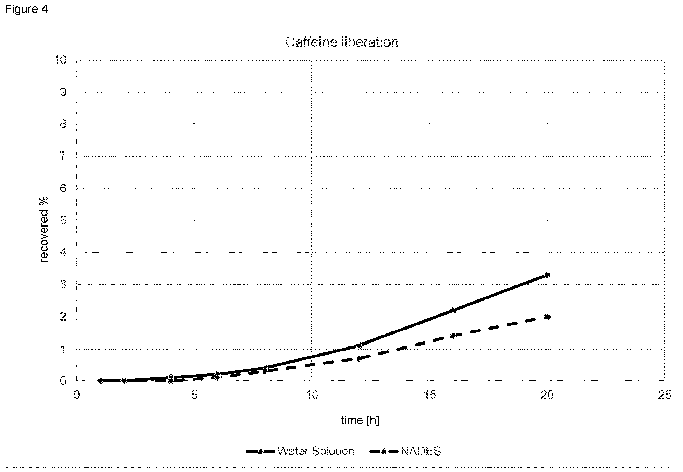

It was shown in figure 4 that the liberation of caffeine over time is surprisingly lower in eutectic solvents according to the invention when compared to the water solution. This is believed to be the reason behind, also surprisingly, its lower dermal absorption.

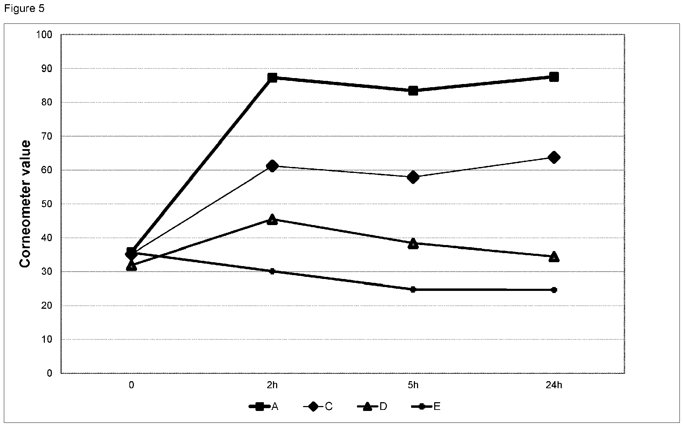

The same phenomena were studied in a W/O emulsion compared to a Nades-in-oil emulsion, both containing 0.5% caffeine, shown in figure 5.

A similar trend was observed with the use of the active molecule Thiamidol.

The eutectic solvents according to the invention shows a surprising reservoir effect. Typically, when actives dissolved in the aqueous phase, actives tend to lose their bioactivity whenever water evaporates, e.g. due to their crystallization. By dissolving the actives of interest in the new eutectic solvents, an increase in the availability could be observed, leading to a better dermal absorption.

Due to the hydrophilic nature of the new solvents, a remarkable superior in vitro moisturization was measured, e.g. for an emulsion containing Betaine : Urea (+15% wt. water) as the internal phase of an emulsion when compared to a standard formula (Nivea Soft). The analysis was made in pig skin disks using a corneometer to measure moisture.

**table 2: corneometer figure 5**

| | sample |
|---|---|
| A | Betaine + Urea (2:3) in water in oil emulsion |
| C | Proline + Urea (2:3) in water in oil emulsion |
| D | Nivea SOFT |
| E | Control sample |

The eutectic solvents according to the invention (A, C) shows a long-lasting moisturizing effect (longer than the limit of 24h in vitro) and a positive effect on reducing TEWL (transepidermal water loss).

The inventive compounds were chosen from the group comprising Alanine, Betaine, Choline chloride, Citric acid, Glycerol, Malic acid, Trehalose, Urea, Glucose, 1,3 - Propanediol, Xylitol, Proline, Sorbitol and Glycine.

Alanine is a α-amino acid. It contains an amine group and a carboxylic acid group, both attached to the central carbon atom which also carries a methyl group side chain. Consequently, its IUPAC systematic name is 2-aminopropanoic acid, and it is classified as a nonpolar, aliphatic α-amino acid. Melting point of Alanine is 258°C.

### Structure of Alanine:

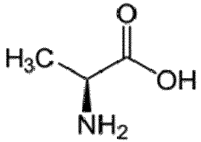

Betaine is a quaternary ammonium compound with three methylgroups and structure

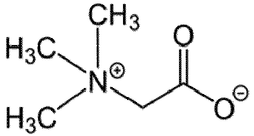

Melting point of betaine is 301 °C.

Choline chloride is an organic compound with the formula (CH3)3NCH2CH2OH]Cl. It is bifunctional, containing both quaternary ammonium salt and an alcohol. The cation is choline, which occurs naturally. It is a white, water soluble salt used mainly in animal feed.

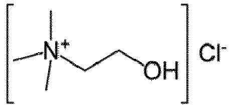

Melting point of choline chloride is 302°C.

Citric acid is a weak organic acid. It occurs naturally in citrus fruits.

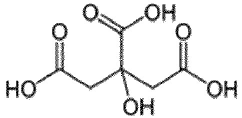

Melting point of citric acid is 156°C.

Glycerol (Glycerin, Propane -1,2,3 -triol) is a common cosmetic ingredient.

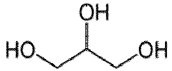

Melting point of glycerol is 18°C.

Malic acid is an organic compound. It is a dicarboxylic acid that is made by all living organisms, contributes to the sour taste of fruits, and is used as a food additive.

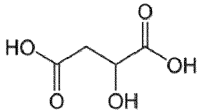

Melting point of malic acid is 130°C.

Trehalose is a disaccharide that consists of two a, a'-1,1-glycosidically linked glucose molecules. Therefore, its systematic name is 1-a-glucopyranosyl-1-a-glucopyranoside.

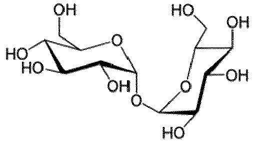

Melting point is between135 ° C and 216°C, depending on the types of anomers.

Urea, chemically the diamide of carbonic acid, is an organic compound with the structure

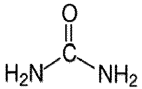

Melting point of urea is 132,5 °C.

Glucose (Glc) is a naturally occurring chemical compound. D-glucose is also referred to as dextrose. D-glucose is the most common monosaccharide.

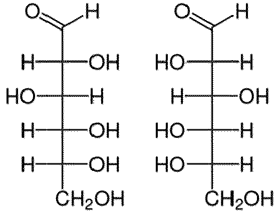

Melting point of glucose is 146°C.

1,3- Propanediol belongs to the group of dihydric alcohols, diols.

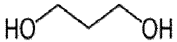

Melting point of 1,3-propandiol is -26°C.

Xylitol is a stereoisomer of the sugar alcohol pentane pentene. As a food additive it bears the name (E 967) and serves as a sugar substitute.

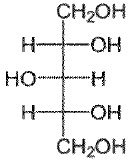

Melting point of xylitol is 94°C.

Proline (L-proline,(S) -pyrrolidine-2-carboxylic acid) is a non-essential proteinogenic heterocyclic secondary α-amino acid.

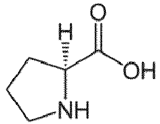

Melting point of proline is 210°C (racemate).

Sorbitol (sorbit, also glucitol or hexanehexol) is one of the alditols (sugar alcohols) and is used in many industrially produced foods (food additive E 420) as a sugar substitute, carrier and humectant.

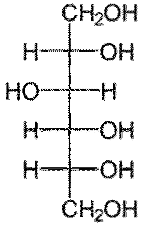

Melting point of Sorbitol is 95°C.

Glycine is the smallest and simplest α-amino acid.

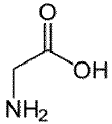

Melting point of glycine is 232-236 °C.

The preferred inventive compounds and molar ratios are:
Betaine : Urea in molar ratios varying from 1:1 to 1:2, esp. 1 : 1,5,
Betaine : Glycerol in molar ratios from 1:3 to 1:4,
Proline : Urea in molar ratios from 1:1 to 1:2,
Proline : Sorbitol in molar ratio 1:1,
Betaine : Sorbitol in molar ratio 1:1,
Betaine : Glucose in molar ratio 1:1 and
preferred with amounts of water from 5 to 40% wt.

**table 3: preferred molar ratio of inventive compounds:**

| Component A | Component B | mol (A) | mol(B) |
|---|---|---|---|
| Alanine | Malic Acid | 1 | 1 |
| Betaine | Malic Acid | 1 | 1 |
| Betaine | Trehalose | 4 | 1 |
| Betaine | Glucose | 4 | 1 |
| Betaine | Glucose | 1 | 1 |
| Betaine | Urea | 2 | 3 |
| Choline chloride | Glucose | 1 | 4 |
| Choline chloride | 1,3-propanediol | 1 | 3 |
| Choline chloride | Urea | 1 | 2 |
| Citric acid | Trehalose | 2 | 1 |
| Citric acid | Xylitol | 1 | 1 |
| Citric acid | Proline | 1 | 1 |
| Glycerol | Betaine | 1 | 1 |
| Glycerol | Betaine | 3 | 1 |
| Glycerol | Glycine | 3 | 1 |
| Glycerol | Citric acid | 2 | 1 |
| Malic Acid | Proline | 1 | 1 |
| Malic acid | Choline Chloride | 1 | 2 |
| Trehalose | Choline Chloride | 1 | 1 |
| Urea | Choline Chloride | 2 | 1 |
| Proline | Urea | 1 | 2 |
| Proline | Urea | 2 | 3 |
| Proline | Sorbitol | 1 | 1 |

Following is shown some examples for actives solubilization:
- Dihydromyricetin
   ∘ Solubility in Water 0.09% wt.
   ∘ Solubility in Betaine:Urea 2:3 > 0.5% wt
   ∘ Solubility in Betaine:Glucose 1:1 > 0.5% wt
   ∘ Solubility in Betaine:Malic Acid 1:1 > 0.5% wt
   ∘ Solubility in Proline:Sorbitol 1:1 > 0.5% wt
   ∘
- Folic Acid
   ∘ Solubility in Water 0.00016% wt.
   ∘ Solubility in Betaine:Glycerol 1:3 > 1% wt
- Thiamidol
   ∘ Solubility in Water 0.017% wt.
   ∘ Solubility in Betaine:Glycerol 1:3 > 2% wt

These examples show the surprising and improved possibilities to incorporate active ingredients into cosmetic compositions.

The inventive composition comprising one or more of an eutectic solvents is preferably a part of a cosmetic composition, especially part of a cosmetic composition based on a water in oil emulsion.

As the comparable test shows, the inventive composition could be used for improving the incorporation of active ingredients into cosmetic compositions and also for improving the stability of active ingredients in cosmetic compositions and the availability of active ingredients from cosmetic compositions.

In addition, the inventive composition comprising antiperspirant active ingredients could be used for improving the anti-perspirant activity compared to a composition without the NADES solvents.

Preferably, the inventive composition comprises on or more antiperspirant active ingredients, especially Calcium-lactate, Magnesiumchloride and/or aluminium salts.

The inventive composition could be used for an improved moisturization of the skin.

## Claims

1. An eutectic solvent formed from the mixture of two different components selected from the group consisting of Alanine, Betaine, Choline chloride, Citric acid, Glycerol, Malic acid, Trehalose, Urea, Glucose, 1,3 - Propanediol, Xylitol, Proline, Sorbitol and Glycine.

2. Solvent according to claim 1, wherein the molar ratio of the two components being chosen in the range of 1 to 4 to 4 to 1.

3. Solvent according to claim 1 or 2 **characterized in that** the eutectic solvent is selected from
Alanine and Malic Acid with Molar ratio of 1 to 1,
Betaine and Malic Acid with Molar ratio of 1 to 1,
Betaine and Trehalose with Molar ratio of 4 to 1,
Betaine and Glucose with Molar ratio of 4 to 1,
Betaine and Glucose with Molar ratio of 1 to 1,
Betaine and Urea with Molar ratio of 3 to 2,
Choline chloride and Glucose with Molar ratio of 1 to 4,
Choline chloride and 1,3-propanediol with Molar ratio of 1 to 3,
Choline chloride and Urea with Molar ratio of 1 to 2,
Citric acid and Trehalose with Molar ratio 2 to 1,
Citric acid and Xylitol with Molar ratio of 1 to 1,
Citric acid and Proline with Molar ratio 1 to 1,
Glycerol and Betaine with Molar ratio of 1 to 1,
Glycerol and Betaine with Molar ratio of 3 to 1,
Glycerol and Betaine with Molar ratio of 4 to 1,
Glycerol and Glycine with Molar ratio of 3 to 1,
Glycerol and Citric acid with Molar ratio of 2 to 1,
Malic Acid and Proline with Molar ratio of 1 to 1,
Malic Acid and Choline Chloride with Molar ratio of 1 to 2,
Trehalose and Choline Chloride with Molar ratio of 1 to 1,
Urea and Choline Chloride with Molar ratio of 2 to 1,
Proline and Urea with Molar ratio of 1 to 2,
Proline and Urea with Molar ratio of 2 to 3 and/or
Proline and Sorbitol with Molar ratio of 1 to 1.

4. A composition comprising one or more of an eutectic solvent according to one of the claims 1 to 3.

5. Composition according to claim 4 as a cosmetic composition.

6. Composition according to claim 5 wherein the cosmetic composition is based on a water in oil emulsion.

7. Composition according to claim 4, 5 or 6 **characterized in that** the eutectic solvent comprises water in an amount up to 40 percent by weight, preferred up to 20 percent by weight, based on the total mass of the eutectic solvent and water.

8. Composition according to one of the claims 4 to 7 comprising one or more active ingredients, especially cosmetic active ingredients.

9. Composition according to claim 8 wherein the active ingredients were chosen from Glycyrrhiza Inflata Root Extract, Silver Citrate, 4-[(Cyclopentylhydroxyphenylacetyl)oxy]-1,1-dimethyl-piperidiniumbromid (GPIP), Isobutylamido Thiazolyl Resorcinol (Thiamidol), Sodium Ascorbyl Phosphate, Folic acid, Tocopherol, Lipoic acid, Kalium-Methoxysalicylate, Vitamin B6 HCI, Pyrus Malus Stem Extract, Glyceryl Glucoside, short and / or long chain hyaluronic acid, 4-Butyl-Resorcin, Octadecene Dioic acid, Caffeine, Carnitine, Creatine, Cystine, I-tyrosine, Alpha-Glucosylrutin, Taurine, Vitamin C/Ascorbic acid, Glycyrrhizic acid, N-Acetylhydroxyprolin, Niacinamide, Magnesium-Ascorbylphosphat, Ellagic acid, licorice root extract, sea salt, Isoserinol, Ascorbylglucosid, Undecenoylphenylalanin, Kojic acid, arbutin, Zingerone, Dihydromyricetin, 4-Hexyl-Resorcin, Phenylethyl Resorcinol, Ubiquinone, esp. Q10, Cyanomethylphenyl Menthan Carboxamid, Menthoxypropanediol, Menthan Carboxamid Ethylpyridin, Hydroxyethylurea, Climbazole, Epsilon-Poly-L-Lysine, Laureth-9, Piroctone Olamine, Selenium sulfide, zinc pyrithione, deodorant and/or antiperspirant active ingredients and plant- and/or fruit extracts.

10. Composition according to claim 8 or 9 wherein the active ingredients are antiperspirant active ingredients, especially Calcium-lactate, Magnesiumchloride and/or aluminium salts.

11. Use of a solvent according to one of the claims 1 to 3 for improving the incorporation of active ingredients into cosmetic compositions.

12. Use of a solvent according to one of the claims 1 to 3 for improving the stability of active ingredients in cosmetic compositions.

13. Use of a solvent according to one of the claims 1 to 3 for improving the availability of active ingredients from cosmetic compositions.

14. Use of a composition according to one of the claims 4 to 9 for an improved moisturization of the skin.

15. Use of a composition according to one of the claims 4 to 10 comprising an antiperspirant active ingredients for improving the anti-perspirant activity.
